# EUROPÄISCHE PATENTSCHRIFT

(11) **EP 1 143 248 B1**
(45) Veröffentlichungstag und Bekanntmachung des Hinweises auf die Patenterteilung: **01.01.2014**
(21) Anmeldenummer: 01104170.4
(22) Anmeldetag: 21.02.2001
(51) Int. Cl.: G01N 33/543, C12Q 1/68, G01N 33/558

(54) **Verfahren zu Immobilisierung von Konjugaten in diagnostischen Tests**
Method to immobilise conjugates in diagnostic tests
Méthode pour l'immobilisation de conjugates dans des épreuves diagnostiques

(30) Priorität: 29.02.2000 DE 10009503
(43) Veröffentlichungstag der Anmeldung: 10.10.2001
(73) Patentinhaber: Roche Diagnostics GmbH, 68305 Mannheim (DE); F. Hoffmann-La Roche AG, 4070 Basel (CH)
(72) Erfinder: Schäffler, Jürgen, Dr., 69469 Weinheim (DE); Upmeier, Barbara, Dr., 82393 Iffeldorf (DE)
(74) Vertreter: Weiss, Wolfgang

(56) Entgegenhaltungen:
- EP-A- 0 833 157
- EP-A- 0 842 949
- WO-A-00/31538
- WO-A-96/38720

## Beschreibung

Die Erfindung betrifft ein Verfahren zum Nachweis eines Analyten in einer Probe unter Verwendung von analytspezifischen Konjugaten, die mindestens eine heterologe Gruppe für eine analytunabhängige Bindung an eine Kontrollzone aufweisen. Weiterhin werden durch die vorliegende Erfindung neue Reagenzienkits bereitgestellt.

Zum Nachweis von Analyten, z.B. Substanzen mit diagnostischer Relevanz, in einer Probe werden analytspezifische Reagenzien, beispielsweise spezifische Bindepartner für den zu bestimmenden Analyten oder/und Analytanaloga verwendet. Bei heterogenen Nachweisverfahren wird zur Bindung des Analyten eine Festphase verwendet, so daß der Analyt zum Zweck des qualitativen oder quantitativen Nachweises von anderen Komponenten abgetrennt werden kann. Diese Festphasenbindung kann durch einen weiteren analytspezifischen Rezeptor vermittelt werden, der vor oder während der Durchführung des Tests an die Festphase gebunden wird. Bei einem Nachweisverfahren nach dem Prinzip des Sandwich-Tests kann ein spezifischer Bindepartner für den Analyten an die Festphase gebunden werden und der Nachweis des Analyten über einen freien, für den Analyten spezifischen Bindepartner erfolgen, der eine Markierungsgruppe trägt. Bei einem kompetitiven Nachweisverfahren kann als festphasenanalytspezifische Reagenz ebenfalls ein spezifischer Binderpartner für den Analyten verwendet werden und der Nachweis des Analyten indirekt durch Bindung eines freien markierten Analytanalogons an die Festphase erfolgen.

Eine besondere technische Ausführungsform von heterogenen Nachweisverfahren sind Teststreifen, die eine definierte Analytnachweiszone für den quantitativen oder qualitativen Nachweis des Analyten enthalten. Der zum Nachweis verwendete freie analytspezifische Rezeptor trägt vorzugsweise eine Direktmarkierung, d.h. eine unmittelbar signalgebende Gruppe, wodurch neben einer gerätegestützten quantitativen Bestimmung auch eine visuelle qualitative Auswertung des Tests möglich ist.

Diagnostische Teststreifen enthalten neben der Analytnachweiszone auch eine Kontrollzone, die dem Anwender eine Differenzierung zwischen negativer Testaussage und unkorrekter Anwendung bzw. Funktionsmangel des Tests ermöglicht. Diese Kontrollzone ist im allgemeinen so ausgestaltet, daß sie eine Immobilisierung des freien analytspezifischen Rezeptors unabhängig von der Anwesenheit des Analyten in der Probe ermöglicht. Somit wird diese Kontrollzone bei korrekter Funktion des Tests in jedem Falle angefärbt.

Die Bindung des freien analytspezifischen Rezeptors in der Kontrollzone kann nach unterschiedlichen Methoden erfolgen. Wenn der freie analytspezifische Rezeptor ein spezifischer Bindepartner für den Analyten ist, kann in der Kontrollzone der Analyt, ein Analytanalogon oder ein Epitop des Analyten immobilisiert werden, wodurch der freie Analytbindeparter aufgefangen wird. Handelt es sich bei dem freien Bindepartner um einen Antikörper, kann für die Kontrollzone daher ein mit dem Antikörper reaktives Antigen, Antigenanalogon oder ein Epitop des Antigens verwendet werden.

Diese Ausführungsform der Kontrollzone ist in einigen Fällen jedoch nicht realisierbar, da der Analyt nicht ausreichend verfügbar ist oder/und nicht genügend charakterisiert ist, um Analoga oder/und Epitope davon herzustellen oder/und der spezifische Bindepartner Regionen des Analyten erkennt, die als Epitop nicht abbildbar oder nach Immobilisierung auf dem Teststreifen nicht mehr zugänglich sind.

In solchen Fällen wurde bisher ein gegen eine homologe Nicht-Analytbinderegion des Bindepartners gerichteter Rezeptor eingesetzt, um den freien analytspezifischen Bindepartner im Kontrollfeld des Teststreifens abzufangen. Wenn der Bindepartner ein Antikörper ist, wurden als Kontrollrezeptoren Anti-Antikörper, die die konstante Region des Nachweisantikörpers erkennen, oder andere für Immunglobuline spezifisch bindefähige Reagenzien, z.B. Protein A oder Protein G, verwendet.

Um eine valide Aussage über das Ergebnis eines heterogenen Nachweisverfahrens im Teststreifenformat zu treffen, ist eine signalgebende Reaktion der Kontrollzone wesentlich, da nur auf diese Weise die korrekte Funktion des Teststreifens überprüft werden kann. Die Anfärbung der Kontrollregion alleine ist als negatives Ergebnis eines funktionstauglichen Tests zu interpretieren, während eine zusätzliche Anfärbung der Analytnachweiszone für das positive Testresultat, im allgemeinen für den Nachweis der Anwesenheit eines Analyten in der getesteten Probe steht.

Das Bindeereignis, welches die Anfärbung der Kontrollzone sicherstellt, kann jedoch aus verschiedenen Gründen gestört sein. Beispielsweise kann durch hohe Analytkonzentrationen in der Probe die Kapazität des freien Analytbindepartners weitgehend erschöpft sein, so daß er mit einem in der Kontrollzone immobilisierten Analyten bzw. einem Epitop oder einem Analogon davon nicht oder nicht mehr ausreichend bindefähig ist. Die Kontrollzone färbt sich dann nur noch schwach oder gar nicht mehr an (Hook-Effekt).

Werden andere von der Analyterkennung des Bindepartners unabhängige Rezeptoren wie z.B. Antikörper-bindende Substanzen, insbesondere Anti-Antikörper als Abfangreagenzien in der Kontrollregion des Teststreifens eingesetzt, so ist das Risiko eines Funktionsverlusts durch den Hook-Effekt vermindert. Dieses Konzept der Teststreifenkontrolle weist jedoch andere gravierende Nachteile auf. So werden als Proben in vielen Fällen biologische Materialien wie Blut, Plasma oder Serum eingesetzt. Dem Fachmann ist bekannt, daß diese Materialien für Antikörper bindefähige Substanzen enthalten, die in einem immunologischen Test mit Antikörpern als spezifische Fang- und Nachweisreagenzien Störungen mit der Folge falschpositiver oder falschnegativer Ergebnisse verursachen können. Um derart bedingte Fehlmessungen vorzubeugen, werden in der Regel oftmals unspezifische Immunglobuline derjenigen Tierspezies als Entstörkomponenten zugesetzt, aus der die spezifischen Nachweisantikörper stammen. Die Menge dieser entstörenden Antikörper übersteigt dabei die Menge der analytspezifischen Antikörper um ein Vielfaches. Sie sind mit den gegen die Nachweisantikörper gerichteten Abfangreagenzien der Kontrollregion des Teststreifens bindefähig und konkurrieren so mit dem Nachweisreagenz um Bindestellen. Entsprechend werden geringere Mengen an Nachweisreagenz fixiert, die Färbung der Kontrollzone fällt deutlich schwächer aus oder findet überhaupt nicht statt.

WO 96/38720 offenbart eine chromatographische Assayvorrichtung zur Detektion und/oder Bestimmung eines Analyten in einen kompetitiven immunoassay.

EP 0 833 157 B1 offenbart Reagenzien, welche in Immunoassays eingesetzt werden können und Vorrichtungen zur Verwendung dieser Reagenzien.

Die der vorliegenden Erfindung zugrundeliegende Aufgabe bestand darin, heterogene Nachweisverfahren bereitzustellen, bei denen Kontrollzonen verwendet werden, welche die oben geschilderten Nachteile des Standes der Technik nicht aufweisen.

Die vorliegende Erfindung wird durch die Ansprüche definiert.

Diese Aufgabe wird gelöst durch Verwendung von derivatisierten freien analytspezifischen Rezeptoren, d.h. es werden ein oder mehrere heterologe Strukturelemente eingeführt, die zuvor im freien Rezeptor nicht vorhanden waren und die unabhängig sind von seiner Markierung und seiner Eigenschaft, den Analyten oder einen Analytbindepartner spezifisch zu binden. Der derivatisierte Rezeptor kann aufgrund des neu eingeführten Strukturelements von einem dagegen gerichteten Rezeptor erkannt und gebunden werden. Dieser weitere Rezeptor wird in der Kontrollzone der für einen heterogenen Test verwendeten Festphase immobilisiert, so daß es möglich ist, den markierten freien Rezeptor in dieser Zone abzufangen. Die Abfangreaktion wird durch die Analytmenge in der Probe nicht beeinflußt.

Das heterologe Strukturelement, welches die Rezeptorbindung ermöglicht, kann so gewählt werden, daß andere mit der Rezeptorbindung interferierende Reaktionen, z.B. durch Entstörsubstanzen weitgehend ausgeschlossen sind.

In einem ersten Aspekt betrifft die vorliegende Erfindung somit ein Verfahren zum Nachweis eines Analyten in einer Probe nach Anspruch 1.

Das erfindungsgemäße Verfahren ist insbesondere für Festphasen geeignet, die ein poröses Material umfassen, wie etwa saugfähige Teststreifen. Darüber hinaus ist das erfindungsgemäße Verfahren jedoch auch für andere Arten von Festphasen geeignet, z.B. nichtporöse Trägermaterialien in Form von Array-Anordnungen, die neben einer oder mehreren Analytbindezonen auch räumlich definierte Kontrollzonen aufweisen.

Die Analytnachweiszone der Festphase ist vorzugsweise so ausgestaltet, daß auf ihr ein analytspezifischer Rezeptor, z.B. ein mit dem Analyten spezifisch bindefähigen Antikörper oder ein Antikörperfragment, immobilisiert ist. Die Immobilisierung kann vor oder während der Testdurchführung erfolgen, vorzugsweise über hochaffine Wechselwirkungen wie Streptavidin bzw. Avidin/Biotin. Andererseits kann die Immobilisierung auch adsorptiv oder durch kovalente Bindung erfolgen.

Bei einem Testverfahren nach dem Sandwichprinzip wird die Bindung des Analyten an die Festphase durch Verwendung eines freien Rezeptors nachgewiesen, der einen für den Analyten spezifischen Bindepartner umfaßt, z.B. einen weiteren gegen den Analyten gerichteten Antikörper. Bei einem kompetitiven Testverfahren kann als freier Rezeptor ein Analytanalogon verwendet werden. Der freie Rezeptor ist vorzugsweise direkt oder indirekt markiert. Bei einer direkten Markierung trägt der freie Rezeptor eine signalgebende Gruppe und bei einer indirekten Markierung eine mit einer signalgebenden Gruppe bindefähige Gruppe. Beispiele für geeignete signalgebende Gruppen sind radioaktive Markierungen, Enzyme, fluoreszierende - bzw. lumineszierende Gruppen oder Farbmarkierungen. Besonders bevorzugt sind Direktmarkierungen und darunter durch optische Methoden nachweisbare Gruppen wie etwa Gold- oder andere Metallpartikel, Farbstoff- oder Fluoreszenzpartikel, z.B. Latexpartikel, oder Silikatpartikel etc.

Im Unterschied zu bekannten Verfahren wird gemäß vorliegender Erfindung ein freier Rezeptor verwendet, der an mindestens ein "heterologes Strukturelement" gebunden ist, das zur Bindung an eine Kontrollzone dient. Unter einem "heterologen Strukturelement" ist im Sinne der vorliegenden Erfindung ein Strukturelement zu verstehen, das in nichtnatürlicher Weise mit dem Rezeptor assoziiert ist. Das heterologe Strukturelement kann direkt an den Rezeptor gebunden sein. Die Bindung zwischen heterologem Strukturelement kann jedoch auch indirekt, z.B. über einen Träger, vermittelt sein.

Das heterologe Strukturelement ist verschieden von der für die analytspezifischen Bindegruppe des freien Rezeptors und auch verschieden von der Markierungsgruppe des freien Rezeptors, sofern eine solche vorhanden ist. Das heterologe Strukturelement ist ein Hapten, vorzugsweise ein niedermolekulares (MW ≤ 3000) Hapten, ein Peptid mit bis zu 25 Aminosäuren oder Biotin. Das heterologe Strukturelement kann kovalent oder durch nichtkovalente Wechselwirkungen an den freien Rezeptor gebunden werden. Bevorzugt ist eine kovalente Bindung.

Das heterologe Strukturelement kann beispielsweise eine durch eine chemische Derivatisierungsreaktion an den freien Rezeptor angefügte Gruppe sein, z.B. ein an ein Polypeptid oder eine Nukleinsäure gekoppeltes Hapten. Diese Derivatisierungsreaktion kann vor oder nach dem Einführen der Markierungsgruppen erfolgen. Üblicherweise wird für die Derivatisierungsreaktion das heterologe Strukturelement in Form eines aktivierten Derivats, z.B. als ein Aktivester wie etwa ein N-Hydroxysuccinimidester oder als Maleimid mit dem freien Rezeptor in Kontakt gebracht, wobei eine Bindung an freie Amino- oder Thiolgruppen des Rezeptors erfolgt.

Der freie Rezeptor kann ausgewählt werden aus Peptiden, Polypeptiden, Glykoproteinen, Lipoproteinen, Nukleinsäuren, Nukleinsäureanaloga, Sacchariden, Polysacchariden und anderen biologischen Substanzen vorzugsweise ist er ein Peptid oder Polypeptid, beispielsweise ein Antikörper. Ein solcher Antikörper kann beispielsweise mit einem Hapten, z.B. Digoxigenin oder Fluorescein, derivatisiert und anschließend adsorptiv an eine Markierungsgruppe, z.B. kolloidale Metall-, beispielsweise Goldpartikel, gekoppelt werden. Ein solcher zweifach-modifizierter Antikörper wird dann in Kombination mit einer Kontrollzone eingesetzt, die immobilisierte Anti-Digoxigenin-Antikörper enthält.

In einer weiteren Ausführungsform des erfindungsgemäßen Verfahrens kann die Bindung des heterologen Strukturelements an den freien Rezeptor über einen Träger vermittelt sein, vorzugsweise über Koimmobilisierung, d.h. sequenzielle oder/und gleichzeitige Immobilisierung auf einem Partikel, z.B. durch Adsorption. Die Partikel werden vorzugsweise aus den bereits zuvor genannten Direktmarkierungen, z.B. Metall-, Farbstoff- oder Fluoreszenzpartikeln ausgewählt. An diese Partikel können im Verlaufe des Herstellungsprozesses nicht allein der freie Rezeptor, sondern noch weitere Begleitsubstanzen gebunden werden, beispielsweise Stabilisatoren, wie etwa Rinderserumalbumin, unspezifische Antikörper (z.B. IgG) oder Netzmittel. Gemäß vorliegender Erfindung kann nun das heterologe Strukturelement in eine dieser Begleitsubstanzen eingeführt werden, so daß ein Konjugat resultiert, das den freien Rezeptor und das heterologe Strukturelement in Form zweier unterschiedlicher, an einem Partikel coimmobilisierter Moleküle umfaßt. Dieses Konjugat erfüllt einerseits die Funktion eines markierten freien Rezeptors und kann andererseits über einen gegen das Strukturelement gerichteten Bindepartner abgefangen werden.

Das erfindungsgemäße Verfahren kann zum Nachweis diagnostisch relevanter Analyten in biologischen Proben eingesetzt werden. Beispiele für biologische Proben sind Körperflüssigkeiten wie Blut, Serum, Plasma, Urin, Speichel und Sperma, Gewebeproben, Proben aus Zellkulturen etc. Die zu testende Probe wird unter geeigneten Bedingungen mit dem Testreagenz und der Festphase in Kontakt gebracht, wobei die Reihenfolge des Inkontaktbringens der einzelnen Komponenten im allgemeinen nicht kritisch ist. Bei Verwendung von Teststreifen liegt das Testreagenz im allgemeinen in trockener Form auf dem Teststreifen vor und wird durch den Kontakt mit der flüssigen Probe aufgelöst. Die Flüssigkeit wandert dann anschließend aufgrund der Kapillarwirkung des saugfähigen Teststreifenmaterials zur Analytnachweis- und Kontrollzone, wo dann das Testergebnis abgelesen werden kann.

Das erfindungsgemäße Verfahren ist grundsätzlich für alle Arten von Nachweissystemen des Typs X/Anti-X geeignet, wo ein Analyt X aufgrund einer hochaffinen spezifischen Wechselwirkung mit einem analytbindefähigen Rezeptor Anti-X nachgewiesen werden kann. Vorzugsweise ist das Verfahren ein immunologisches Nachweisverfahren oder ein Nukleinsäure-Hybridisierungsverfahren. Selbstverständlich sind jedoch auch andere Nachweisverfahren wie etwa Zucker/Lectin-Nachweisverfahren geeignet.

Die Verwendung von freien analytspezifischen Rezeptoren, die zusätzlich ein heterologes Strukturelement zur Bindung an eine Kontrollzone tragen, eignet sich insbesondere für Nachweisverfahren, bei denen Entstörungsreagenzien anwesend sein müssen, um das häufige Auftreten von falschen Ergebnissen ausschließen zu können. Beispiele für solche Entstörungsreagenzien sind mit dem freien Rezeptor strukturell verwandte, aber nicht analytspezifische Substanzen, z.B. Antikörper aus derselben Spezies oder/und derselben Immunglobulinklasse. Gerade in solchen Fällen kann die vorliegende Erfindung die einzige Möglichkeit zur Bereitstellung einer funktionsfähigen Kontrollzone sein.

Noch ein weiterer Gegenstand der vorliegenden Erfindung ist ein Reagenzienkit nach Anspruch 18.

Vorzugsweise trägt der freie Rezeptor eine signalgebende Gruppe oder eine mit einer signalgebenden Gruppe bindefähige Gruppe, besonders bevorzugt trägt der freie Rezeptor eine direkt nachweisbare signalgebende Gruppe. Der erfindungsgemäße Reagenzienkit enthält weiterhin einen analytspezifischen Festphasenrezeptor, der auf der Analytnachweiszone in immobilisierter Form vorliegen kann oder der eine auf der Analytnachweiszone, beispielsweise über eine hochaffine Wechselwirkung wie etwa Streptavidin/Biotin immobilisierbare Festphasenbindegruppe enthält. Die im Reagenzienkit enthaltene Festphase ist vorzugsweise ein poröses Material, insbesondere ein saugfähiger Teststreifen. Weiterhin kann der Reagenzienkit Entstörsubstanzen enthalten, die mit dem freien Rezeptor eine strukturelle Ähnlichkeit besitzen.

Weiterhin wird die vorliegende Erfindung durch die nachfolgenden Abbildungen und Beispiele ausführlich erläutert. Es zeigen:

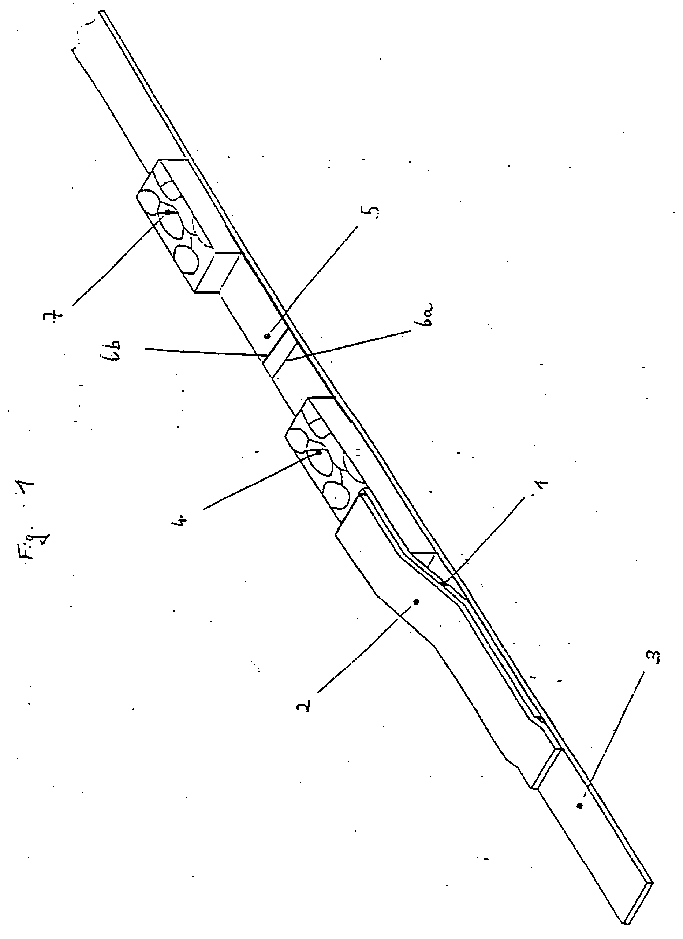

- Abb. 1: die schematische Darstellung eines Teststreifens. Der Teststreifen enthält eine wasserfeste Trägerfolie (3), auf der ein Trägervlies mit einem markierten Reagenz (1) und ein Trägervlies mit einem immobilisierbaren Reagenz (2) sowie einem Vlies (4) zur Abtrennung fester Probenbestandteile, wie etwa roter Blutzellen, aufgebracht sind. Weiterhin sind auf der Trägerfolie (1) eine Trägermembran (5) mit einer Signalzone (6a) und einer Kontrollzone (6b) aufgebracht. Schließlich enthält der Träger ein Saugvlies (7). Die Trägervliese (2, 1, 4, 5 und 7) sind derart angeordnet, daß sie miteinander in chromatographischem Kontakt stehen. Eine auf das Vlies (2) aufgebrachte Probe strömt über (1), (4) und (5) in das Saugvlies (7);
- Abb. 2: die Entwicklung eines Signalstrichs auf einem D-Dimer-Teststreifen mit Digoxigenin-markiertem Antikörper-Goldkonjugat als Testreagenz;
- Abb.3: die Entwicklung eines Anti-Mausantikörper-Kontrollstrichs auf einem D-Dimer-Teststreifen in Abhängigkeit vom Vorhandensein eines monoklonalen Entstör-Antikörpers (Vergleich);
- Abb. 4: die Entwicklung eines Anti-Digoxigenin-Antikörper-Kontrollstrichs bei einem D-Dimer-Teststreifen in Abhängigkeit vom Vorhandensein eines monoklonalen Entstör-Antikörpers (Erfindung).

### Beispiele

### Beispiel 1: Derivatisierung eines monoklonalen Antikörpers mit aktiviertem Digoxigenin

10 mg eines gereinigten monoklonalen Antikörpers gegen D-Dimer werden mit einer Konzentration von 10 mg/ml in 100 mM Kaliumphosphatpuffer, pH 8,5, gelöst und im Wasserbad auf 25 °C temperiert. Unter Rühren werden langsam 0,065 ml einer Lösung von Digoxigenin-3-O-methylcarbonyl-ε-aminocarbonsäure-N-hydroxy-succinimidester (Roche Diagnostics GmbH, Mannheim, Deutschland) in Dimethylsulfoxid (1,052 mg/ml) hinzugegeben. Der Ansatz wird 90 min unter Rühren bei 25 °C inkubiert. Danach wird der Ansatz mit Lysin-hydrochlorid aufgestockt ad 10 mM unter Beibehaltung des pH-Wertes von pH 8,5. Nach weiterer 15-minütiger Inkubation bei 25 °C wird das Material fließend gegen das 5000fache Volumen 30 mM Kaliumphosphatpuffer, pH 7,6, 100 mM Natriumchlorid dialysiert.

### Beispiel 2: Herstellung eines Nachweisreagenz für einen immunologischen Schnelltest durch Immobilisierung eines Digoxigenin-markierten monoklonalen Antikörpers an kolloidales Gold

500 ml kolloidales Gold mit einem durchschnittlichen Partikeldurchmesser von 20 nm (optische Dichte 1,0 bei 520 nm) werden auf Raumtemperatur temperiert und über einen Cellulosenitratfilter mit einer Porengröße von 0,2 mm filtriert. Durch Zusatz von Kaliumcarbonat wird ein pH-Wert von pH 7,6 eingestellt.

Zum Filtrat werden 50 ml einer Lösung des Digoxigenin-markierten Antikörpers gegen D-Dimer (wie in Beispiel 1 hergestellt) mit einer Konzentration von 0,1 mg/ml Tris/HCl-Puffer (2 mM Tris/HCl, pH 7,6, 20 mM Natriumchlorid) hinzugegeben. Nach 30-minütiger Inkubation unter leichtem Rühren wird der Ansatz mit Rinderserumalbumin ad 1 % (w/v) aufgestockt und weitere 30 min gerührt. Danach wird das Material mittels Tangentialfluß-Ultrafiltration über Membranen mit einer Ausschlußgrenze von 30.000 Dalton aufkonzentriert, bis die optische Dichte (gemessen von 520 nm) 20 beträgt. Das Antikörper-Goldkonjugat wird durch Zugabe von Saccharose ad 4 % (w/v) und Natriumazid ad 0,095 % (w/v) stabilisiert.

### Beispiel 3: Immunologischer Schnelltest

### a) Konjugatträger

Als poröser Träger für die Goldkonjugatimprägnierung wird ein Mischvlies, bestehend aus einer Du Pont Mischesterfaser, Zellwolle und dem Naßreißverfestiger Kuralon im Gewichtsverhältnis 80:20:20 verwendet. Die Herstellung des Vlieses erfolgt analog der Beschreibung in EP-A-0 326 135. Das Vlies (Flüssigkeitsaufnahme etwa 30 µl/cm²) wird mit einer Lösung getränkt, die 1 ml der goldkonjugierten Digoxigenin-markierten Antikörper-Lösung aus Beispiel 2 und 4 ml HEPES-Puffer (130 mM HEPES/Natriumhydroxid, pH 7,5) enthält. Anschließend wird getrocknet und auf eine Breite von 18 mm aufgeschnitten.

### b) Konjugatträger mit biotinyliertem Antikörper

Ein gereinigter zweiter monoklonaler Antikörper gegen D-Dimer wird mit D-Biotinoyl-ε-aminocapronsäure-N-hydroxysuccinimidester (Roche Molecular Biochemicals) analog zu Beispiel 1 mit Biotin derivatisiert.

Ein weiteres wie unter 3a) beschriebenes Mischvlies wird mit einer Lösung getränkt, die 10 µg/ml des mit Biotin konjugierten zweiten Antikörpers und 10 mg/ml Rinderserumalbumin in HEPES-Puffer (130 mM HEPES/Natriumhydroxid, pH 7,5) enthält. Anschließend wird getrocknet und auf eine Breite von 20 mm aufgeschnitten.

### c) Teststreifen

Mit diesen Reagenzvliesen, d.h. Goldkonjugatvlies (1) und Vlies mit biotinyliertem Antikörper (2), werden Teststreifen gemäß Abb. 1 durch Aufsiegeln auf eine Trägerfolie (3) aus Polystyrol (Melinex) mittels eines Schmelzkleberstriches aufgebaut. Zur Abtrennung roter Blutzellen wird ein mittels 10 Gewichtsanteilen Kuralon verfestigtes Glasfaservlies (4) verwendet. Die Herstellung des Vlieses erfolgt analog der Beschreibung in EP-B-0 239 002. Das Glasfaservlies hat typischerweise ein Flächengewicht von ca. 180 g/cm², eine Dicke von ca. 1,5 mm und eine Tränkaufnahme von ca. 1.400 ml/m². Das Vlies wird mit einem 80 mM 2-Morpholinethansulfonsäure-Puffer pH 5,6 getränkt, der 0,2 Gew % Rinderserumalbumin und 0,1 Gew % n-Octylglucosid enthält. Das getrocknete Vlies wird auf eine Breite von ca. 12 mm aufgeschnitten. Auf eine Cellulosenitratmembran (5) (Sartorius SN 11301, 145 *µ*m Dicke, 8 *µ*m Porendurchmesser) werden über Nadeldosierung (Kanülendurchmesser 0,16 mm) je eine Lösung, die 4,5 mg/ml (poly-)Streptavidin (Roche Molecular Biochemicals) in destilliertem Wasser enthält, für die Erzeugung eines Signalstriches (6a), und 5 mg/ml polyklonale Antikörper vom Schaf gegen Maus-Antikörper (PAK < Maus >, Roche Molecular Biochemicals) in destilliertem Wasser oder 1 mg/ml polyklonale Antikörper vom Schaf gegen Digoxigenin (PAK<Dig>, Roche Molecular Biochemicals) in PBS, für die Erzeugung eines Kontroll-Striches (6b) aufgebracht. Die Lösungen werden so aufgebracht, daß Striche (6a, 6b) mit einer Breite von ca. 0,5 mm entstehen. Die Cellulosenitratmembran wird anschliessend längs der Strichrichtung auf eine Breite von 15 mm geschnitten. Am entgegengesetzten Ende der Membran wird ein "Saugvlies" (7) angebracht. Als Saugvlies wird ein ungetränktes, mit 5 Gewichtsanteilen Kuralon verfestigtes Glasfaservlies verwendet. Die Herstellung des Vlieses erfolgt analog der Beschreibung in EP-B-O 239 002. Das Vlies hat typischerweise ein Flächengewicht von 180 g/m² und eine Dicke von 1,5 mm. Zum Einbau in den Testträger wird es auf 7 mm Breite aufgeschnitten. Der gesiegelte Testträger wird anschließend zu einzelnen Teststreifen von 6 mm Breite geschnitten.

### d) Messung

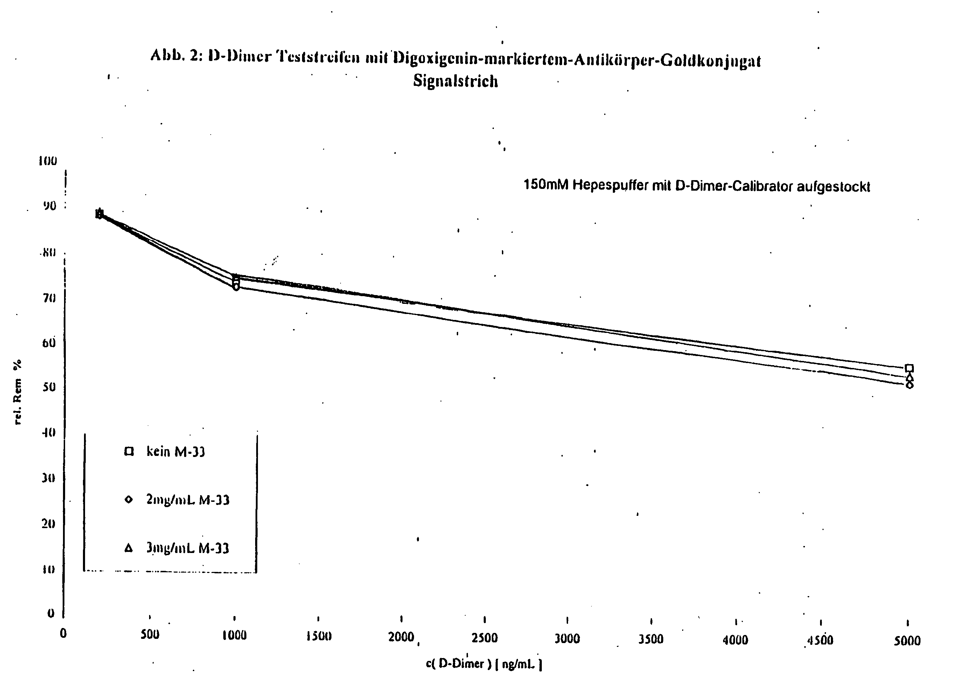

Die Probe (150 µl Vollblut, Plasma oder ähnliches) wird auf die Konjugatvliese aufgegeben, wobei sie die Konjugate anlöst. In der Probe vorhandener Analyt bildet Sandwichkomplexe mit den beiden Konjugat-Antikörpern aus. Diese Lösung wird durch Kapillarkräfte durch das Glasfaservlies, wo gegebenenfalls Erythrozyten abgetrennt werden, und die Membran zum Saugvlies transportiert. Am (poly-)Streptavidin-Strich werden biotinylierte Antikörper und gebildete Sandwichkomplexe abgefangen. In Abhängigkeit von der gebildeten Sandwichkomplex-Konzentration wird ein mehr oder weniger intensiver roter Strich, verursacht durch das gebundene kolloidale Gold, gebildet. Am Kontrollstrich (PAK < Maus > oder PAK < Dig >) wird überschüssiges Goldkonjugat durch Bindung des monoklonalen Maus-Antikörpers bzw. der Digoxigenin-Markierung abgefangen. An dieser Strichposition kann auch bei Abwesenheit von Analyt die ordnungsgemäße Funktion des Testes durch eine Rotfärbung erkannt werden. Nach 10 Minuten Reaktionszeit wird die Intensität der Striche mit Hilfe einer CCD-Kamera vermessen. Abb. 2 zeigt die Entwicklung des Signalstriches in Abhängigkeit von der Analytkonzentration (rel. Rem % = Reflexion des Striches bezogen auf die ungefärbte Membran rechts und links des Striches).

### Beispiel 4: "HAMA-entstörte" Teststreifen

Im Biotinkonjugat-Vlies nach Beispiel 3 werden steigende Mengen eines monoklonalen HAMA-(humane Anti-Maus-Antikörper)-Entstörantikörpers (MAK-33, Roche Molecular Biochemicals) zum Abfangen von Anti-Maus-Antikörpern, die in der zu analysierenden Probe vorhanden sein können, zur Tränkrezeptur gegeben.

Die Teststreifen werden wie unter Beispiel 3 beschrieben hergestellt.

Eine Konzentrationsreihe von analythaltigen Lösungen wird wie unter Beispiel 3 beschrieben vermessen.

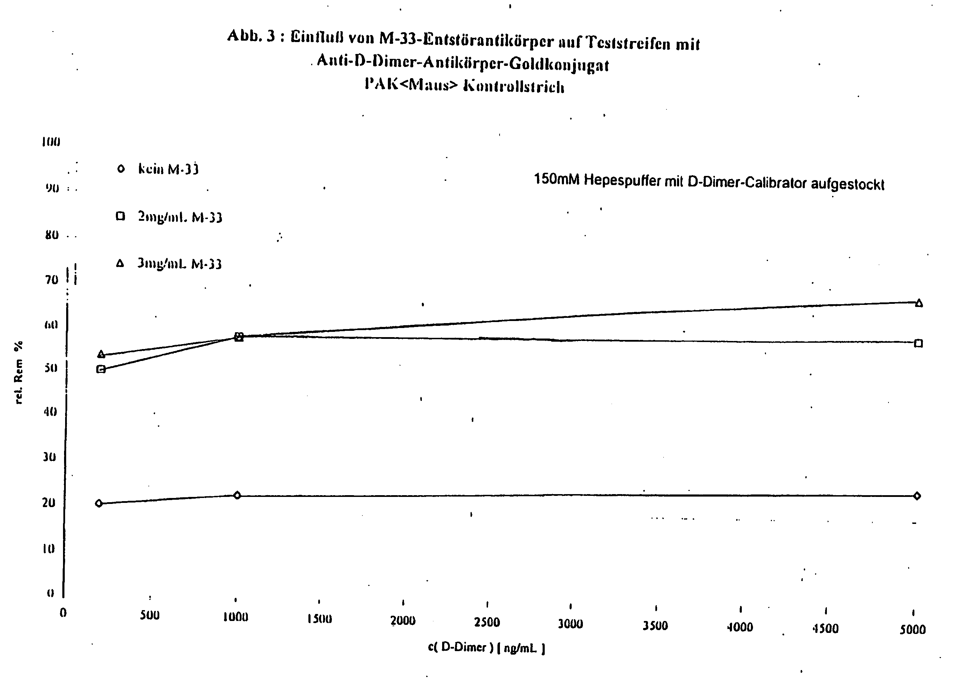

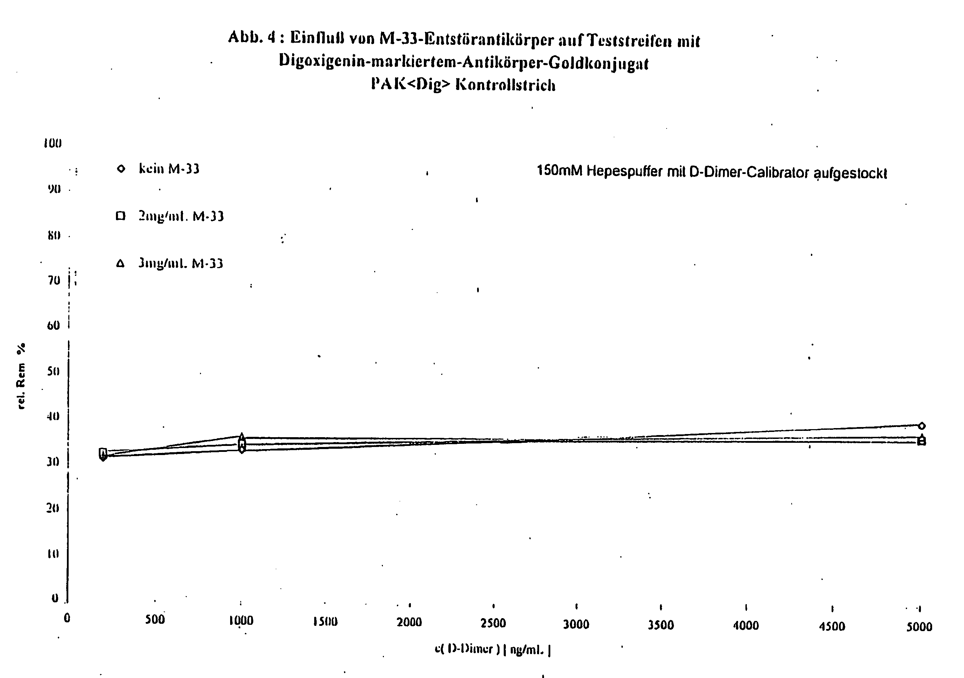

Abb. 3 zeigt die Ausbildung des Kontrollstriches beim Einsatz unterschiedlicher Mengen Entstör-Antikörper beim Einsatz von PAK <Maus> (rel. Rem % = Reflexion des Strichs bezogen auf die ungefärbte Membran rechts und links des Strichs). Die Intensität des Strichs wird mit steigender Menge Entstörantikörper deutlich geringer. Abb. 4 zeigt die Ausbildung des Kontrollstrichs beim Einsatz unterschiedlicher Mengen Entstörantikörper beim Einsatz von PAK<Dig>. Die Menge an Entstörantikörper hat keinen erkennbaren Einfluß auf die Ausbildung des Kontrollstrichs.

## Patentansprüche

1. Verfahren zum Nachweis eines Analyten in einer Probe,
umfassend die Schritte:
(a) Bereitstellen einer Festphase, umfassend eine Analytnachweiszone und eine Kontrollzone, wobei die Analytnachweiszone für den quantitativen oder/und qualitativen Nachweis des Analyten in der Probe und die Kontrollzone für den Nachweis der Funktionsfähigkeit des Testreagenz vorgesehen ist,
(b) Inkontaktbringen der Festphase mit der Probe und dem Testreagenz, wobei das Testreagenz einen freien analytspezifischen Rezeptor und mindestens ein heterologes Strukturelement für eine spezifische Bindung an die Kontrollzone enthält, wobei das heterologe Strukturelement unabhängig von den analytspezifischen Eigenschaften des Rezeptors ist und wobei das heterologe Strukturelement ausgewählt wird aus Haptenen, Peptiden mit bis zu 25 Aminosäuren oder Biotin, wobei der freie Rezeptor an mindestens ein heterologes Strukturelement gebunden ist, und
(c) Bestimmen des Vorhandenseins oder/und der Menge des Analyten in der Probe über die Analytnachweiszone und Überprüfen der Testfunktion über die Kontrollzone, wobei in der Kontrollzone ein Rezeptor oder Bindepartner immobilisiert ist, der für das heterologe Strukturelement spezifisch ist.

2. Verfahren nach Anspruch 1,
**dadurch gekennzeichnet,**
**daß** die Analytnachweiszone einen analytspezifischen immobilisierten Rezeptor enthält.

3. Verfahren nach einem der Ansprüche 1 oder 2,
**dadurch gekennzeichnet,**
**daß** der freie Rezeptor eine signalgebende Gruppe oder eine mit einer signalgebenden Gruppe bindefähige Gruppe trägt.

4. Verfahren nach Anspruch 3,
**dadurch gekennzeichnet,**
**daß** die signalgebende Gruppe eine durch optische Methoden nachweisbare Gruppe, insbesondere ausgewählt aus Metall-, Farbstoff- und Fluoreszenzpartikeln, z.B. Latexpartikeln und Silikatpartikeln, umfaßt.

5. Verfahren nach einem der vorhergehenden Ansprüche,
**dadurch gekennzeichnet,**
**daß** die Festphase ein poröses Material umfaßt.

6. Verfahren nach Anspruch 5,
**dadurch gekennzeichnet,**
**daß** die Festphase als saugfähiger Teststreifen ausgebildet ist.

7. Verfahren nach einem der Ansprüche 1 bis 4,
**dadurch gekennzeichnet,**
**daß** die Festphase eine Array-Anordnung umfaßt.

8. Verfahren nach einem der vorhergehenden Ansprüche,
**dadurch gekennzeichnet,**
**daß** das heterologe Strukturelement durch kovalente Bindung mit dem freien Rezeptor gekoppelt ist.

9. Verfahren nach einem der vorhergehenden Ansprüche,
**dadurch gekennzeichnet,**
**daß** das heterologe Strukturelement für die Kontrollzone durch eine Derivatisierungsreaktion in den freien Rezeptor eingeführt wird.

10. Verfahren nach einem der Ansprüche 1 bis 9,
**dadurch gekennzeichnet,**
**daß** der freie Rezeptor und das heterologe Strukturelement auf einem Partikel coimmobilisiert werden.

11. Verfahren nach einem der vorhergehenden Ansprüche,
**dadurch gekennzeichnet,**
**daß** der freie Rezeptor einen für den Analyten spezifischen Bindepartner umfaßt.

12. Verfahren nach einem der vorhergehenden Ansprüche 1 bis 11,
**dadurch gekennzeichnet,**
**daß** der freie Rezeptor ein Analytanalogon umfaßt.

13. Verfahren nach einem der vorhergehenden Ansprüche,
**dadurch gekennzeichnet,**
**daß** der freie Rezeptor ausgewählt wird aus Peptiden, Polypeptiden, Glykoproteinen, Lipoproteinen, Nukleinsäuren, Nukleinsäureanaloga, Sacchariden, Polysacchariden und anderen biologischen Substanzen.

14. Verfahren nach einem der vorhergehenden Ansprüche,
**dadurch gekennzeichnet,**
**daß** der freie Rezeptor ausgewählt wird aus Antikörpern und Antikörperfragmenten.

15. Verfahren nach einem der vorhergehenden Ansprüche,
**dadurch gekennzeichnet,**
**daß** ein Testreagenz verwendet wird, das weiterhin mit dem freien Rezeptor strukturell verwandte, aber nicht analytspezifische Substanzen enthält.

16. Verwendung des Verfahrens nach einem der vorhergehenden Ansprüche zum Nachweis diagnostisch relevanter Analyten in biologischen Proben.

17. Verwendung nach Anspruch 16 in einem immunologischen Nachweisverfahren oder einem Nukleinsäure-Hybridisierungsverfahren.

18. Reagenzienkit zum Nachweis eines Analyten in einer Probe umfassend
(a) eine Festphase, umfassend eine Analytnachweiszone und eine Kontrollzone, wobei die Analytnachweiszone für den quantitativen oder/und qualitativen Nachweis der Analyten in der Probe und die Kontrollzone für den Nachweis der Funktionsfähigkeit des Testreagenz vorgesehen ist, wobei auf der Analytnachweiszone ein immobilisierter oder immobilisierbarer analyt-spezifischer Rezeptor enthalten ist, wobei in der Kontrollzone ein Rezeptor oder Bindepartner immobilisiert ist, der für das heterologe Strukturelement spezifisch ist,
(b) ein Testreagenz umfassend einen freien analytspezifischen Rezeptor, wobei der freie analytspezifische Rezeptor ein Antikörper ist und mindestens ein heterologes Strukturelement für eine spezifische Bindung an die Kontrollzone, wobei das heterologe Strukturelement unabhängig von den analytspezifischen Eigenschaften des Rezeptors ist, wobei der freie Rezeptor an mindestens ein heterologes Strukturelement gebunden ist, wobei das heterologe Strukturelement ausgewählt wird aus Haptenen, Peptiden mit bis zu 25 Aminosäuren oder Biotin,
(c) eine nicht-analytspezifische Substanz, wobei es sich um einen Antikörper aus derselben Spezies oder/und derselben Immunoglobulinklasse wie der freie Rezeptor, handelt.

19. Reagenzienkit nach Anspruch 18,
**dadurch gekennzeichnet,**
**daß** der freie Rezeptor eine signalgebende Gruppe oder eine mit einer signalgebenden Gruppe bindefähige Gruppe trägt.

20. Reagenzienkit nach einem der Ansprüche 18 oder 19,
**dadurch gekennzeichnet,**
**daß** die Festphase ein poröses Material umfaßt.

21. Verwendung eines Reagenzienkits nach einem der Ansprüche 18 bis 20 in einem Verfahren zum Nachweis eines Analyten.

22. Verwendung nach Anspruch 21 in einem Verfahren nach einem der Ansprüche 1 bis 15.

## Claims

1. Method for detecting an analyte in a sample,
comprising the steps:
(a) preparing a solid phase, comprising an analyte detection area and a control area, in which the analyte detection area is provided for detecting the analyte in the sample quantitatively and/or qualitatively and the control area is provided for detecting the efficiency of the test reagent,
(b) bringing the solid phase into contact with the sample and the test reagent, in which the test reagent contains a free, analyte-specific receptor and at least one heterologous structural element for specific bonding to the control area, in which the heterologous structural element is independent of the analyte-specific properties of the receptor and in which the heterologous structural element is selected from haptenes, peptides with up to 25 amino acids or biotin, in which the free receptor is bonded to at least one heterologous structural element, and
(c) determining the presence and/or quantity of the analyte in the sample through the analyte detection area and checking the test function through the control area, in which a receptor or bonding partner is immobilised in the control area, which is specific for the heterologous structural element.

2. Method according to claim 1,
**characterised in that**
the analyte detection area contains an analyte-specific, immobilised receptor.

3. Method according to one of claims 1 or 2,
**characterised in that**
the free receptor has a group giving a signal or a group, which may be bonded to a group giving a signal.

4. Method according to claim 3,
**characterised in that**
the group giving a signal comprises a group, which may be detected by optical methods, particularly selected from metal, dye or fluorescent particles, such as latex particles and silicate particles for example.

5. Method according to one of the previous claims,
**characterised in that**
the solid phase comprises a porous material.

6. Method according to claim 5,
**characterised in that**
the solid phase is made as an absorbent test strip.

7. Method according to one of claims 1 to 4,
**characterised in that**
the solid phase comprises an array arrangement.

8. Method according to one of the previous claims,
**characterised in that**
the heterologous structural element is coupled to the free receptor by covalent bonding.

9. Method according to one of the previous claims,
**characterised in that**
the heterologous structural element for the control area is introduced into the free receptor through a derivatisation reaction.

10. Method according to one of claims 1 to 9,
**characterised in that**
the free receptor and the heterologous structural element are co-immobilised on a particle.

11. Method according to one of the previous claims,
**characterised in that**
the free receptor comprises a bonding partner, which is specific for the analyte.

12. Method according to one of previous claims 1 to 11,
**characterised in that**
the free receptor comprises an analyte analog.

13. Method according to one of the previous claims,
**characterised in that**
the free receptor is selected from peptides, polypeptides, glycoproteins, lipoproteins, nucleic acids, nucleic acid analogs, saccharides, polysaccharides and other biological substances.

14. Method according to one of the previous claims,
**characterised in that**
the free receptor is selected from antibodies and antibody fragments.

15. Method according to one of the previous claims,
**characterised in that**
a test reagent is used, which also contains substances, which are structurally related to the free receptor, but not analyte-specific.

16. Use of the method according to one of the previous claims for detecting diagnostically relevant analytes in biological samples.

17. Use according to claim 16 in an immunological detection method or a nucleic acid hybridisation method.

18. Reagent kit for detecting an analyte in a sample, comprising
(a) a solid phase, comprising an analyte detection area and a control area, in which the analyte detection area is provided for detecting the analyte in the sample quantitatively and/or qualitatively and the control area is provided for detecting the efficiency of the test reagent, in which an analyte-specific receptor, which is immobilised or may be immobilised, is contained in the analyte detection area, which is specific for the heterologous structural element,
(b) a test reagent, comprising a free, analyte-specific receptor, in which the free analyte-specific receptor is an antibody and at least one heterologous structural element for specific bonding to the control area, in which the heterologous structural element is independent of the analyte-specific properties of the receptor, in which the free receptor is bonded to at least one heterologous structural element, in which the heterologous structural element is selected from haptenes, peptides with up to 25 amino acids or biotin,
(c) a non-analyte-specific substance, in which it is an antibody from the same species and/or the same immunoglobulin class as the free receptor.

19. Reagent kit according to claim 18,
**characterised in that**
the free receptor has a group giving a signal or a group, which may be bonded to a group giving a signal.

20. Reagent kit according to one of claims 18 or 19,
**characterised in that**
the solid phase comprises a porous material.

21. Use of a reagent kit according to one of claims 18 to 20 in a method for detecting an analyte.

22. Use according to claim 21 in a method according to one of claims 1 to 15.

## Revendications

1. Procédé pour la détection d'un analyte dans un échantillon, comprenant les étapes :
(a) mise à disposition d'une phase solide, comprenant une zone de détection d'un analyte et une zone de contrôle, la zone de détection d'un analyse étant prévue pour la détection quantitative et/ou qualitative de l'analyte dans l'échantillon et la zone de contrôle étant prévue pour la détection de l'aptitude au fonctionnement du réactif de test,
(b) mise en contact de la phase solide avec l'échantillon et le réactif de test, le réactif de test contenant un récepteur libre spécifique à l'analyte et au moins un élément de structure hétérologue pour une liaison spécifique à la zone de contrôle, l'élément de structure hétérologue étant indépendant des propriétés spécifiques à l'analyte du récepteur et l'élément de structure hétérologue étant choisi parmi les haptènes, les peptides comprenant jusqu'à 25 acides aminés ou la biotine, le récepteur libre étant lié à au moins un élément de structure hétérologue, et
(c) détermination de la présence et/ou de la quantité de l'analyte dans l'échantillon via la zone de détection de l'analyte et test de la fonction de test via la zone de contrôle, un récepteur ou un partenaire de liaison qui est spécifique à l'élément de structure hétérologue étant immobilisé dans la zone de contrôle.

2. Procédé selon la revendication 1, **caractérisé en ce que** la zone de détection de l'analyte contient un récepteur immobilisé spécifique à l'analyte.

3. Procédé selon l'une quelconque des revendications 1 ou 2, **caractérisé en ce que** le récepteur libre porte un groupe émettant un signal ou un groupe apte à la liaison avec un groupe émettant un signal.

4. Procédé selon la revendication 3, **caractérisé en ce que** le groupe émettant un signal comprend un groupe pouvant être détecté par des procédés optiques, en particulier choisi parmi les particules métalliques, de colorant et fluorescentes, par exemple des particules de latex et de silicate.

5. Procédé selon l'une quelconque des revendications précédentes, **caractérisé en ce que** la phase solide comprend un matériau poreux.

6. Procédé selon la revendication 5, **caractérisé en ce que** la phase solide est réalisée sous forme de bandelette test absorbante.

7. Procédé selon l'une quelconque des revendications 1 à 4, **caractérisé en ce que** la phase solide comprend une disposition en série.

8. Procédé selon l'une quelconque des revendications précédentes, **caractérisé en ce que** l'élément de structure hétérologue est couplé au récepteur libre par liaison covalente.

9. Procédé selon l'une quelconque des revendications précédentes, **caractérisé en ce que** l'élément de structure hétérologue pour la zone de contrôle est introduit dans le récepteur libre par une réaction de dérivation.

10. Procédé selon l'une quelconque des revendications 1 à 9, **caractérisé en ce que** le récepteur libre et l'élément de structure hétérologue sont co-immobilisés sur une particule.

11. Procédé selon l'une quelconque des revendications précédentes, **caractérisé en ce que** le récepteur libre comprend un partenaire de liaison spécifique à l'analyte.

12. Procédé selon l'une quelconque des revendications précédentes 1 à 11, **caractérisé en ce que** le récepteur libre comprend un analogue de l'analyte.

13. Procédé selon l'une quelconque des revendications précédentes, **caractérisé en ce que** le récepteur libre est choisi parmi les peptides, les polypeptides, les glycoprotéines, les lipoprotéines, les acides nucléiques, les analogues d'acide nucléique, les saccharides, les polysaccharides et d'autres substances biologiques.

14. Procédé selon l'une quelconque des revendications précédentes, **caractérisé en ce que** le récepteur libre est choisi parmi les anticorps et les fragments d'anticorps.

15. Procédé selon l'une quelconque des revendications précédentes, **caractérisé en ce qu'**on utilise un réactif de test qui contient en outre des substances structurellement apparentées au récepteur libre mais non spécifiques à l'analyte.

16. Utilisation du procédé selon l'une quelconque des revendications précédentes pour la détection d'analytes pertinents en diagnostic dans des échantillons biologiques.

17. Utilisation selon la revendication 16 dans un procédé de détection immunologique ou un procédé d'hybridation d'acide nucléique.

18. Kit de réactifs pour la détection d'un analyte dans un échantillon, comprenant
(a) une phase solide, comprenant une zone de détection d'un analyte et une zone de contrôle, la zone de détection d'un analyse étant prévue pour la détection quantitative et/ou qualitative de l'analyte dans l'échantillon et la zone de contrôle étant prévue pour la détection de l'aptitude au fonctionnement du réactif de test, un récepteur immobilisé ou immobilisable, spécifique à l'analyte, étant contenu dans la zone de détection de l'analyte, un récepteur ou un partenaire de liaison spécifique à l'élément de structure hétérologue étant immobilisé dans la zone de contrôle,
(b) un réactif de test comprenant un récepteur libre spécifique à l'analyte, le récepteur libre spécifique à l'analyte étant un anticorps et au moins un élément de structure hétérologue pour une liaison spécifique à la zone de contrôle, l'élément de structure hétérologue étant indépendant des propriétés spécifiques à l'analyte du récepteur, le récepteur libre étant lié à au moins un élément de structure hétérologue, l'élément de structure hétérologue étant choisi parmi les haptènes, les peptides comprenant jusqu'à 25 acides aminés ou la biotine,
(c) une substance non spécifique à l'analyte, où il s'agit d'un anticorps de la même espèce et/ou de la même classe d'immunoglobulines que le récepteur libre.

19. Kit de réactifs selon la revendication 18, **caractérisé en ce que** le récepteur libre porte un groupe émettant un signal ou un groupe apte à la liaison avec un groupe émettant un signal.

20. Kit de réactifs selon l'une quelconque des revendications 18 ou 19, **caractérisé en ce que** la phase solide comprend un matériau poreux.

21. Utilisation d'un kit de réactifs selon l'une quelconque des revendications 18 à 20 dans un procédé pour la détection d'un analyte.

22. Utilisation selon la revendication 21 dans un procédé selon l'une quelconque des revendications 1 à 15.
